# EUROPÄISCHE PATENTANMELDUNG

(11) **EP 2 452 638 A1**
(43) Veröffentlichungstag der Anmeldung: **16.05.2012**
(21) Anmeldenummer: 10405220.4
(22) Anmeldetag: 15.11.2010
(51) Int. Cl.: A61B 17/34, A61D 7/00

(54) **Vorrichtung zur Feststoffinjektion**

(71) Anmelder: forteq Nidau AG, 2560 Nidau (CH)
(72) Erfinder: Schmalz, Christian, 3250 Lyss (CH)
(74) Vertreter: Rüfenacht, Philipp Michael

(57) **Zusammenfassung**

Eine Vorrichtung (1) zum Injizieren eines Feststoffes (600) in einen menschlichen oder tierischen Körper, insbesondere eine Spritze für die Injizierung eines Feststoffmedikaments, umfassend einen in einem Gehäuse (200) verfahrbar gelagerter Spritzenkörper (100), verbunden mit einer Kanüle (122). Der Spritzenkörper (100) ist in proximaler Richtung mit einer Federkraft beaufschlagt, wobei die Bewegung des Spritzenkörpers (100) in proximaler Richtung mittels einer ersten, mit dem Spritzenkörper (100) zusammenwirkenden Verriegelungsvorrichtung (121, 220) entriegelbar ist. Ein primärer Stössel (400), welcher im Spritzenkörper (100) und in der Kanüle verfahrbar ist umfasst einen Anschlag (410), welcher mit dem Spritzenkörper (100) zusammenwirken kann, wobei der Anschlag (410) in einem Zustand unmittelbar vor der Entriegelung der ersten Verriegelungsvorrichtung (121, 220) vom Spritzenkörper (100) proximal beabstandet ist. Über den Anschlag (410) des primären Stössels (400) kann derselbe durch den Spritzenkörper (100) bei entriegelter ersten Verriegelungsvorrichtung (121, 220) proximal zurückgefahren werden. Die erste Verriegelungsvorrichtung (121, 220) ist dabei durch ein an der Vorrichtung (1) proximal angeordnetes Betätigungselement (500) deaktivierbar.

## Beschreibung

Die Erfindung betrifft eine Vorrichtung zum Injizieren eines Feststoffes in einen menschlichen oder tierischen Körper, umfassend einen in einem Gehäuse verfahrbar gelagerter Spritzenkörper, welcher mit einer Kanüle verbunden ist, wobei der Spritzenkörper in proximaler Richtung mit einer Federkraft beaufschlagt ist, und wobei die Bewegung des Spritzenkörpers in proximaler Richtung mittels einer ersten, mit dem Spritzenkörper zusammenwirkenden Verriegelungsvorrichtung entriegelbar ist und wobei die Vorrichtung weiter einen primären Stössel umfasst, welcher im Spritzenkörper und in der Kanüle verfahrbar ist, wobei der primäre Stössel einen Anschlag umfasst, welcher mit dem Spritzenkörper zusammenwirken kann, wobei der Anschlag in einem Zustand unmittelbar vor der Entriegelung der ersten Verriegelungsvorrichtung vom Spritzenkörper proximal beabstandet ist; und wobei über den Anschlag des primären Stössels derselbe durch den Spritzenkörper bei entriegelter ersten Verriegelungsvorrichtung proximal zurückgefahren werden kann.

### Stand der Technik

Die Injektion von Feststoffen ist ein gängiges Verfahren bei der Langzeitmedikation (Depot-Injektion). Dazu wird ein Depot im Körper abgelegt, welches über eine vorbestimmte Zeitdauer, im Idealfall in konstanter und kontinuierlicher Weise, das Medikament an den Körper abgibt. Typische Anwendungen der Langzeitmedikation sind Schmerztherapien, Hormontherapien für die Empfängnisverhütung, etc. Weiter wird die Injektion von Feststoffen auch im Zusammenhang mit subkutanen Injektionen von Chips, zum Beispiel Mikroprozessoren oder Speicherchips, angewandt.

Eine Injektion eines Feststoffes erfolgt typischerweise, indem der Feststoff durch einen Stössel in einer im Körper befindlichen Kanüle gehalten wird, worauf die Kanüle bei ortsfestem Stössel zurückgezogen wird und so den Feststoff im Körper hinterlässt.

Die WO 99/42148 A2 (LG Chemical LTD; 20.02.1998) zeigt eine Spritzenvorrichtung für Tiere, welche eine Schutzhülse und ein Gehäuse umfasst. Eine Nadel, in welcher das zu injizierende Material gehalten ist, ist fix mit einer Nadelhalterung verbunden, welche im Gehäuse verfahrbar gelagert und in proximaler Richtung mit einer Federkraft beaufschlagt ist. Die Nadelhalterung ist über eine Verriegelungsvorrichtung im distalen Ende gehalten. Die Nadel wird in den Körper geführt. Die Verriegelungsvorrichtung wird durch Einpressen des distalen Endes des Gehäuses entriegelt, worauf sich die Feder entspannt und den Spritzenkörper mit der Kanüle in einer ersten Phase bei ortsfestem Stössel und in einer zweiten Phase mit dem Stössel zusammen zurückfährt.

Die NL 8901124 A (Nedap; 03.05.1989) zeigt eine Vorrichtung zur Injektion eines Feststoffes mit einem Gehäuse und einem Betätigungselement, welches fix mit dem Stössel verbunden ist. Vor der Betätigung befindet sich die Hohlnadel im Gehäuse. Wird nun das Betätigungselement nach unten geführt, so wird die Hohlnadel mit dem Stössel entgegen einer Feder, welche dabei gespannt wird, in den Körper geführt. Nach der vollständigen Einführung werden Halteelemente des Betätigungselements zum Halten der Hohlnadel auseinandergepresst, worauf sich die Feder entspannt und die Hohlnadel bei ortsfestem Stössel zurück in das Gehäuse gefahren wird.

Die WO 99/42148 A2 und die NL 8901124 A haben den Nachteil, dass der Feststoff in genau einer bestimmten Eindringtiefe der Nadel platziert wird. Weiter ist das Eindrücken des Gehäuses in den Körper für den Patienten als unangenehm zu werten. Wenn die Deaktivierung nicht unmittelbar beim Einführen erfolgt, muss die Vorrichtung mit zusätzlicher Kraft an den Körper gepresst werden, was den Patienten unangenehm sein und Schmerzen verursachen kann. Ist aber die Verriegelungsvorrichtung zu sensibel eingestellt, so kann sie sich bei unsachgemässer Handhabung leicht zu früh entriegeln. Dies kann mitunter sehr unangenehm sein, wenn die Kanüle bereits teilweise im Körper eingeführt ist und damit der Feststoff nur teilweise im Körper zu liegen kommt. Weiter besteht der Nachteil, dass der Feststoff in der Kanüle gehalten werden muss, bevor die Injektion erfolgt. Entweder muss dazu der Feststoff mit einer Kraft beaufschlagt sein, welche denselben beschädigen kann, oder es muss in Kauf genommen werden, dass der Feststoff vor dessen Injektion aus der Kanüle fällt.

### Darstellung der Erfindung

Aufgabe der Erfindung ist es, eine des eingangs genannten technischen Gebiets zugehörender Vorrichtung zur Feststoffinjektion zu schaffen, welche einfach aufgebaut und sicher in der Bedienung ist.

Die Lösung der Aufgabe ist durch die Merkmale des Anspruchs 1 definiert. Gemäss der Erfindung ist die erste Verriegelungsvorrichtung durch ein an der Vorrichtung proximal angeordnetes Betätigungselement deaktivierbar.

Der Begriff "distal" bezeichnet im Folgenden eine Richtung zum Körper hin, in welchen der Feststoff injiziert werden soll, und der Begriff "proximal" bezeichnet die dazu entgegengesetzte Richtung, respektive vom Körper weg. In Relation zur Vorrichtung sind die beiden Richtungen als jeweils parallel zu einer Längsachse zu verstehen, wobei die Längsachse zum Beispiel durch das Gehäuse, den Spritzenkörper, die Kanüle oder durch einen der beiden Stössel definiert werden kann. Falls nicht anders gekennzeichnet sind die Richtungen "distal" und "proximal" als parallel zu einer Längsachse des primären Stössels zu verstehen.

Unter dem Begriff "Feststoff" wird das in den Körper einzuführende Präparat verstanden. Der Feststoff kann als Feststoffmedikament, als einen eine Flüssigkeit, Gel oder ähnliches, enthaltenden Behälter oder auch als Mikrochip oder ähnliches ausgebildet sein. Die Vorrichtung ist nicht eingeschränkt auf einen spezifischen Feststoff, sonder kann in einem breiten Feld eingesetzt werden, mit der Einschränkung, dass das zu injizierende Präparat zumindest in einer äussersten Schicht nicht flüssig, insbesondere keine zu tiefe Viskosität aufweist. Vorzugsweise ist der zu injizierende Stoff in seiner äussersten Schicht formstabil.

Die Vorrichtung umfasst einen in einem Gehäuse verfahrbar gelagerten Spritzenkörper, welcher mit einer Kanüle verbunden ist. Je nach Ausführungsform können die Kanüle und der Spritzenkörper auch einstückig ausgebildet sein. Insbesondere kann der Spritzenkörper als ein Abschnitt der Kanüle ausgebildet sein. Für die Kanüle kann eine Schutzkappe vorgesehen sein, welche vor der Verwendung der Vorrichtung entfernt wird.

Die verfahrbare Lagerung des Spritzenkörpers im Gehäuse erfolgt typischerweise als Gleitlager, ohne bewegliche Teile. Dem Fachmann sind genügend Techniken bekannt, die Führung des Spritzenkörpers im Gehäuse zu optimieren. Dazu gehören insbesondere die Materialwahl der einzelnen Teile, Wahl der Kontaktflächen und die Oberflächenbeschaffenheit der Teile. Dabei ist besonders darauf zu achten, dass sich der Spritzenkörper im Gehäuse nicht verkanten kann. Natürlich können auch Kugellager oder dergleichen vorgesehen sein, um eine einwandfreie Lagerung des Spritzenkörpers zu gewährleisten, dies ist jedoch aufgrund des hohen Konstruktionsaufwands bestenfalls dann vorzusehen, wenn die Vorrichtung zur mehrfachen Verwendung vorgesehen ist.

Der Spritzenkörper ist in proximaler Richtung mit einer Federkraft beaufschlagt. Die Federkraft wird vorzugsweise durch eine Feder, insbesondere durch eine Spiralfeder bereitgestellt. Vorzugsweise ist die Feder distal zum Spritzenkörper angeordnet und damit als Druckfeder ausgebildet. Damit wird in besonders einfacher Weise eine Rückstellkraft für den Spritzenkörper bereitgestellt. Um die Auslösung der Feder kontrollieren zu können, ist eine Verriegelungsvorrichtung vorgesehen, welche deaktivierbar ausgebildet ist (siehe unten). Damit wird eine für den Anwender besonders ergonomische Vorrichtung zur Injektion eines Feststoffes geschaffen, da die Kanüle nicht von Hand zurückgefahren werden muss. Eine solche Bewegung kann heikel sein, da sie typischerweise in dieselbe Richtung erfolgt, in welcher die Kanüle aus dem Körper gezogen wird. Bei herkömmlichen Injektionsvorrichtungen, bei welchen das Zurückziehen der Kanüle von Hand erfolgt, muss daher besonders darauf geachtet werden, dass die Vorrichtung selbst nicht zurückgezogen wird, da sonst der Feststoff aus dem Körper gezogen werden könnte.

In Varianten können auch andere Elemente eingesetzt werden, welche eine Federkraft bewirken können. Dazu gehören zum Beispiel Zugfedern oder Gummibänder, welche zwischen dem proximalen Ende des Gehäuses und dem Spritzenkörper wirken können, aber auch Fluiddrucksysteme mit einem komprimierbaren Fluid, insbesondere eine Luftdruckpatrone. Je nach Ausführungsform können solche Lösungen jedoch technisch schwieriger zu realisieren sein.

Der primäre Stössel ist im Spritzenkörper und der Kanüle verfahrbar und umfasst einen Anschlag, welcher vor der Entriegelung der ersten Verriegelungsvorrichtung vom Spritzenkörper proximal beabstandet ist. Die Verriegelungsvorrichtung wird nämlich nach dem Einführen der Kanüle, womit der Feststoff im Körper platziert ist, entriegelt, um in einer ersten Phase die Kanüle bei ortsfestem primärem Stössel zurück zu ziehen. In dieser ersten Phase erreicht der Spritzenkörper den Anschlag des primären Stössels und führt diesen in einer zweiten Phase proximal zurück. Unmittelbar vor der ersten Phase, das heisst vor der Entriegelung der ersten Verriegelungsvorrichung ragen der primäre Stössel und die Kanüle über das distale Ende des Gehäuses hinaus, wobei die Kanüle wiederum über das distale Ende des primären Stössels hinausragt. Im Innenraum der Kanüle, vor dem primären Stössel befindet sich in diesem Zustand der Feststoff. Während der ersten Phase, nach der Entriegelung der Verriegelungsvorrichtung wird nun die Kanüle bei ortsfestem primärem Stössel zurückgefahren, wobei der Feststoff durch den ortsfesten primären Stössel im Körper an Ort und Stelle gehalten wird. Am Ende der ersten Phase kontaktiert der Spritzenkörper, welcher zusammen mit der Kanüle verfährt, den Anschlag des primären Stössels. In diesem Zustand ragt der primäre Stössel aus der Kanüle hervor. Dabei ist es unerheblich, ob die Kanüle vollständig im Spritzenkörper liegt oder noch aus diesem hervorragt. In der zweiten Phase wird der primäre Stössel mit dem Spritzenkörper und der Kanüle zusammen weiter in proximaler Richtung verfahren. Am Ende der zweiten Phase befinden sich vorzugsweise die Kanüle und der primäre Stössel innerhalb des Gehäuses, womit ein hygienischer und sicherer Endzustand nach der Injektion der Vorrichtung erreicht wird. Dadurch, dass sich die Kanüle und der primäre Stössel nach der Injektion im Gehäuse befinden, kann für den Anwender ein Verletzungsrisiko durch die Kanüle sowie ein Risiko der Kontamination vermindert werden.

Die erste Verriegelungsvorrichtung ist durch ein an der Vorrichtung proximal angeordnetes Betätigungselement deaktivierbar.

Durch die Ausbildung eines Betätigungselements zum Deaktivieren der Verriegelungsvorrichtung wird erreicht, dass letztere in kontrollierter Weise entriegelt und damit die Kanüle auch kontrolliert zurückgeführt werden kann. Dies hat insbesondere den Vorteil, dass der Anwender auf die Auslösung des Rückführmechanismus vorbereitet ist und dadurch nicht erschrickt.

Des Weiteren ermöglicht die Ausbildung eines Betätigungselements dem Verwender, den Feststoff in einer variablen Tiefe unter der Haut zu platzieren. Dazu kann der Verwender die Kanüle in gewünschter Tiefe in den Körper einführen, so dass der Feststoff an der gewünschten Stelle zu liegen kommt, und anschliessend das Betätigungselement betätigen. In diesem Moment entspannt sich die Feder, womit der Spritzenkörper zusammen mit der Kanüle zurück verfahren wird. Der primäre Stössel bleibt in dieser Phase ortsfest, so dass der Feststoff an Ort gehalten werden kann. Nachdem die Kanüle in dieser ersten Phase zurückgezogen ist, kontaktiert der Spritzenkörper den Anschlag des primären Stössels und verfährt mit diesem zusammen in einer zweiten Phase weiter zurück, bis die Kanüle und der primäre Stössel vollständig im Spritzenkörper zu liegen kommen.

Durch die proximale Anordnung des Betätigungselements an der Vorrichtung wird erreicht, dass die Betätigung in ergonomischer Weise erfolgen kann. Ein Verwender ist sich nämlich gewohnt, eine herkömmliche Spritze am proximalen Ende zu betätigen, womit im die Anordnung des Betätigungselements der Vorrichtung in ergonomischer Weise entgegenkommt.

Vorzugsweise ist vor der Verwendung der Vorrichtung der Feststoff im Spritzenkörper gehalten und mittels des primären Stössels in die Kanüle überführbar. Der Feststoff kann zusätzlich durch eine Rückhaltevorrichtung im Spritzenkörper gehalten sein, so dass dieser nicht vor der Injektion aus der Kanüle rutschen kann. Die Rückhaltevorrichtung kann grundsätzlich in bekannter Weise vorgesehen sein. Bevorzugt ist allerdings eine Rückhaltevorrichtung, welche den Feststoff schonend zurückhält, das heisst, eine, welche den Feststoff nicht, oder nur in geringem Masse mit einer radialen Kraft beaufschlägt, sondern lediglich durch ein Element, welches distal vor dem Feststoff in den Spritzenkörper hineinragt, zurückhält. Dies kann zum Beispiel mittels axial orientierten länglichen federnden Elementen am Spritzenkörper erreicht werden, welche zwei radial nach innen ragende Nasen aufweisen, wobei der Feststoff zwischen den Nasen gehalten ist. In einem proximalen Bereich sind die Elemente federnd und verschwenkbar am Spritzenkörper gehalten. Die Deaktivierung der Rückhaltevorrichtung erfolgt dabei mittels des primären Stössels, welcher beim Einführen in den Spritzenkörper die proximal angeordneten Nasen kontaktiert und damit die Elemente radial nach aussen treibt und so den Feststoff freigibt.

Alternativ kann der Feststoff auch in der Kanüle gehalten sein. In diesem Fall kann es von Vorteil sein, den Feststoff zum Beispiel mittels Reibung vor der Injektion in der Kanüle zurückzuhalten.

Bevorzugt umfasst die Verriegelungsvorrichtung ein federndes Element und ein damit zusammenwirkendes starres Rastelement, welche an einer Aussenseite des Spritzenkörpers und an der Innenseite des Gehäuses so angeordnet sind, dass sie in verriegeltem Zustand ineinander greifen.

Das federnde Element kann selbst aus einem federnden Material gebildet sein, wie zum Beispiel einem federnden Kunststoff, Metall oder dergleichen. Weiter kann es auch einen starren Teil umfassen, welcher mittels eines federnden Teils mit einer Federkraft beaufschlagt wird. Dazu kann ein starres Kunststoffteil, Metallteil oder dergleichen eingesetzt werden, welches mittels einer Feder, insbesondere einer Spiralfeder, Schenkelfeder, Kegelfeder etc., einem federnden Kunststoffteil, zum Beispiel einem Gummiband, mit einer Federkraft beaufschlagt ist. Das Rastelement kann als vorstehendes Element oder als Kerbe ausgebildet sein.

Grundsätzlich kann die Verriegelung auch auf andere, dem Fachmann bekannte Weise erfolgen. Statt eines federnden Elements kann auch ein lediglich verschwenkbares oder verfahrbares Element vorgesehen sein, welches indirekt durch die Feder, welche den Spritzenkörper mit einer Kraft beaufschlägt, durch Kraftschluss, respektive durch eine Klemmwirkung der Feder, gehalten wird. Die Verriegelungsvorrichtung kann auch direkt in die Feder eingreifen.

Das federnde Element ist bevorzugt als längliche, in verriegeltem Zustand bezüglich einer Längsachse verschwenkte Zunge ausgebildet, welche durch eine radial beaufschlagte Kraft im Wesentlichen in die axiale Richtung verschwenkbar ist, wodurch die Verriegelungsvorrichtung entriegelt werden kann. Bevorzugt wird die Federkraft durch die federnde Ausbildung der Zunge selbst bereitgestellt.

Alternativ kann das federnde Element auch als radial nach innen gerichteter federbeaufschlager Stift ausgebildet sein, welcher in eine Kerbe des Spritzenkörpers hineinragt. Dem Fachmann sind auch weitere Ausführungsformen bekannt.

Bevorzugt ist das federnde Element am Gehäuse angeordnet. Damit wird eine einfach ausgebildete Verriegelungsvorrichtung erreicht. Das federnde Element kann an das Gehäuse angeformt sein, oder als separates Element mit dem Gehäuse verbunden werden.

Alternativ kann das federnde Element auch mit dem Spritzenkörper verbunden sein.

Vorzugsweise ist das starre Rastelement als vorstehende Nase ausgebildet, insbesondere am Spritzenkörper angeformt. Der Spritzenkörper kann dazu einstückig mit der Nase ausgebildet sein.

Statt der Nase kann auch das proximale Ende des Spritzenkörpers selbst als Rastelement dienen, wobei die Zungen mit dem Ende des Spritzenkörpers zusammenwirken. Weiter kann auf das starre Rastelement auch verzichtet werden, insbesondere wenn die Zunge direkt mit der Feder zusammenwirkt.

Bevorzugt sind bezüglich einer Längsachse mehrere symmetrisch angeordnete, insbesondere zwei gegenüberliegende erste Verriegelungsvorrichtungen vorgesehen. Damit kann verhindert werden, dass sich der Spritzenkörper im Gehäuse verkantet, wodurch eine robuste Ausführungsform der Rückführvorrichtung der Kanüle erreicht wird.

Falls der Spritzenkörper hinreichend im Gehäuse geführt ist, kann aber auch eine Verriegelungsvorrichtung ausreichen.

Vorzugsweise ist die erste Verriegelungsvorrichtung mittels eines als sekundären Stössel ausgebildeten Betätigungselements deaktivierbar. Damit wird eine besonders benutzerfreundliche Vorrichtung geschaffen, da diese damit nur ein Betätigungselement für das Verfahren des Feststoffes in die Kanüle und die Entriegelung der Verriegelungsvorrichtung benötigt. Damit wird insbesondere auch der Aufbau der Vorrichtung vereinfacht.

Alternativ kann auch ein separates Betätigungselement für die Deaktivierung der Verriegelungsvorrichtung vorgesehen sein. Ein solches Betätigungselement kann zum Beispiel seitlich am Gehäuse angeordnet sein, wobei in diesem Fall die Zungen vorzugsweise mit dem Spritzenkörper verbunden sind und mittels eines radial orientierten Bolzens nach innen verschwenkt werden, so dass sich die Feder entspannen und den Spritzenkörper proximal verfahren kann.

Der sekundäre Stössel ist vorzugsweise als proximal geschlossener Hohlzylinder ausgebildet, welcher im Gehäuse verfahrbar gelagert ist. Damit wird erreicht, dass bei eingefahrenem sekundärem Stössel der Spritzenkörper im sekundären Stössel aufgenommen werden kann. Dies erlaubt eine kompakte Konstruktion der Vorrichtung, insbesondere bezüglich einer Länge der Vorrichtung. Vorzugsweise entspricht die Form des Querschnitts des sekundären Stössels derjenigen des Gehäuses. Die Form des Querschnitts ist vorzugsweise kreisförmig, kann aber auch quadratisch oder oval sein oder sonst eine geeignete Form aufweisen. Bei nicht kreisförmigem Querschnitt kann erreicht werden, dass der sekundäre Stössel verdrehgesichert im Gehäuse verfahren werden kann, was der Ergonomie bei der Verwendung zuträglich sein kann. Der sekundäre Stössel kann auch seitlich axiale Durchbrechungen aufweisen. Innenseitig können Strukturen zur Erhöhung der Stabilität vorgesehen sein. Dem Fachmann sind auch weitere Varianten geläufig.

In Varianten kann der sekundäre Stössel auch als stabförmiges Element mit einem Kopf zur Betätigung vorgesehen sein.

Bevorzugt ist die erste Verriegelungsvorrichtung durch Kontaktierung des federnden Elements durch den in im Gehäuse eingefahrenem Zustand befindlichen sekundären Stössel entriegelbar. Damit wird eine besonders ergonomische Entriegelung der Verriegelungsvorrichtung erreicht, da der Verwender dazu, wie bei einer herkömmlichen Spritze, den Stössel in das Gehäuse einfahren muss. Beim Einfahren des sekundären Stössels kontaktiert dieser mit seinem distalen Ende die Zunge der Verriegelungsvorrichtung und verschwenkt diese in die axiale Richtung, womit die Zunge nicht mehr in Eingriff mit dem starren Rastelement steht. Damit wird der Spritzenkörper nicht mehr zurückgehalten und kann durch die Federkraft proximal verfahren.

Wie obig erwähnt, bestehen auch andere Möglichkeiten zur Entriegelung der Verriegelungsvorrichtung.

Vorzugsweise weist der sekundäre Stössel an einem distalen Ende in distaler Richtung eine kontinuierlich zunehmende Verjüngung auf, womit in eingefahrenem Zustand das federnde Element radial nach aussen geführt ist. Damit wird erreicht, dass der sekundäre Stössel die in distaler Richtung ragenden Zungen erfassen kann, ohne durch diese blockiert zu werden, bevor sie nach aussen geführt sind. Weiter kann damit der sekundäre Stössel so ausgeführt werden, dass er satt im Gehäuse geführt ist, das heisst im Wesentlichen kein Spiel hat. Je nach Ausführungsform, insbesondere wenn der sekundäre Stössel verdrehgesichert im Gehäuse gelagert ist, kann die Verjüngung auch nur lokal an diesen Stellen vorgesehen sein, wo ein Kontakt mit den Zungen auftreten kann. Bei verdrehgesichertem sekundärem Stössel kann auch lediglich eine, bis zum distalen Ende ragende Einbuchtung vorgesehen sein, sofern dadurch das Zurückführen des Spritzenkörpers nicht behindert wird.

Alternativ kann auf die Verjüngung auch verzichtet werden. In diesem Fall kann es von Vorteil sein, wenn hinter den Zungen im Gehäuse eine Ausnehmung zur Aufnahme der Zunge vorgesehen ist.

Bevorzugt ist der sekundäre Stössel lösbar mit dem primären Stössel verbunden. Dadurch, dass der sekundäre Stössel mit dem primären Stössel verbunden ist, wird erreicht, dass der primäre Stössel, welcher den Feststoff in die Kanüle überführt, zusammen mit dem sekundären Stössel, insbesondere mittels des sekundären Stössels betätigt werden kann. Dies vereinfacht die Bedienung der Vorrichtung. Vorzugsweise ist der primäre Stössel vom proximalen Ende des sekundären Stössels beabstandet lösbar gehalten. Damit wird erreicht, dass in gelöstem Zustand der primäre Stössel innerhalb des sekundären Stössels proximal verfahrbar ist. Damit kann beim Zurückfahren des Spritzenkörpers die zweite Phase, bei welcher der primäre Stössel zusammen mit dem Spritzenkörper verfährt, ermöglicht werden.

In Varianten kann der sekundäre Stössel auch fest mit dem primären Stössel verbunden, oder auch einstückig ausgebildet sein. In diesem Fall würde aber in der zweiten Phase der Rückführung der Kanüle mit dem sekundären Stössel auch der primäre Stössel proximal verfahren werden. In dieser Phase müsste der Verwender besonders darauf achten, dass er die proximale Bewegung des sekundären Spritzenkörpers nicht behindert.

Der sekundäre Stössel weist vorzugsweise innenseitig, im proximalen Bereich federnde Rasthaken auf, welche als zweite Verriegelungsvorrichtung, zum Verriegeln des Anschlages des primären Stössels, ausgebildet sind. Vorzugsweise weist der primäre Stössel entsprechende Elemente auf, welche mit den federnden Rasthaken zusammenwirken können. Damit wird eine besonders einfache zweite Verriegelungsvorrichtung geschaffen.

In Varianten kann der primäre Stössel auch mittels einer perforierten Verbindung, respektive über eine Sollbruchstelle mit dem sekundären Stössel verbunden sein. Dem Fachmann sind auch weitere Möglichkeiten bekannt, wie der primäre Stössel lösbar am sekundären Stössel gehalten werden kann.

Bevorzugt ist die zweite Verriegelungsvorrichtung durch den Spritzenkörper entriegelbar. Während der Rückführung der Kanüle ist der Spritzenkörper das der Verriegelungsvorrichtung am nächsten stehende bewegliche Element. Damit wird eine besonders einfache Entriegelung der zweiten Verriegelungsvorrichtung erreicht. Zum Entriegeln kontaktiert und betätigt der Spritzenkörper am Ende der ersten Phase die federnden Rasthaken und löst so die zweite Verriegelungsvorrichtung, worauf der Spritzenkörper zusammen mit dem primären Stössel proximal verfahren kann.

In Varianten kann die zweite Verriegelungsvorrichtung auch durch ein separates Betätigungselement von Hand entriegelt werden.

Vorzugsweise ist der Anschlag als ein radial vom primären Stössel abstehendes Element, insbesondere als radial orientierte Kreisscheibe, ausgebildet. Dadurch, dass das abstehende Element rotationssymmetrisch ist, wird insbesondere bei nicht verdrehgesichertem sekundärem Stössel, gewährleistet, dass die Rasthaken auch beim Verdrehen des sekundären Stössels in Eingriff mit dem abstehenden Element stehen. So kann verhindert werden, dass der primäre Stössel mitdrehen muss und möglicherweise beschädigt wird. Die federnden Rasthaken sind bevorzugt als axial orientierte und radial verschwenkbare Zungen ausgebildet, welche innenseitig Ausnehmungen zur Aufnahme des Anschlages aufweisen. Damit kann der kreisscheibenförmige Bereich in einfacher Weise aufgenommen werden. Die Zungen sind dazu über das proximale Ende mit dem Bodenbereich und im proximalen Bereich seitlich mit der Innenwand des als einseitig geschlossenen sekundären Stössels verbunden oder damit einstückig ausgebildet. Die radial verschwenkbaren Zungen sind bevorzugt durch den Spritzenkörper radial nach aussen treibbar, womit die zweite Verriegelungsvorrichtung gelöst werden kann. Dazu sind die Zungen in einem distalen Bereich geringfügig von der Innenwand des sekundären Stössels beabstandet. Vorzugsweise sind mindestens zwei gegenüberliegende Zungen, besonders bevorzugt drei regelmässig angeordnete Zungen, vorgesehen. Es können aber auch mehr als drei, oder bei genügend stabil geführtem primärem Stössel auch genau eine Zunge vorgesehen sein kann.

Der Anschlag kann grundsätzlich eine beliebige Form aufweisen. Zum Beispiel kann es von Vorteil sein, wenn der Anschlag bei einem Einsatz von mehr als zwei Zungen als entsprechendes n-Eck ausgebildet ist, wobei jede Zunge eine Seite in Eingriff nimmt. Durch den geraden Bereich des Anschlags kann damit ein sicherer Eingriff gewährleistet werden. Andere Formen, wie zum Beispiel eine ovale oder eine geeignete polygonale Form, sind auch nicht ausgeschlossen.

Bevorzugt umfasst das Gehäuse in einem proximalen Bereich einen umlaufenden Flansch oder zwei gegenüberliegende radial nach aussen ragende Griffelemente. Damit wird eine Bedienung der Vorrichtung analog zu einer herkömmlichen Spritze ermöglicht. Dies ist insbesondere dann von Vorteil, wenn - gemäss einer bevorzugten Ausführungsform - der sekundäre Stössel das einzige Betätigungselement darstellt. In der Anwendung kann der Verwender die beiden Flansche mit Zeige- und Mittelfinger hintergreifen und mit dem Daumen den sekundären Stössel einfahren. In einer bevorzugten Variante weist das proximale Ende des sekundären Stössels weiter einen radial nach aussen stehenden Vorsprung auf, wodurch ein Sicherungselement zwischen dem Vorsprung und den Flanschen eingesetzt werden kann. Eine lösbare Fixierung kann zum Beispiel über eine Clipbefestigung am sekundären Stössel erfolgen. Das Sicherungselement gewährleistet, dass vor der Verwendung der sekundäre Stössel nicht versehentlich in das Gehäuse eingefahren wird. Dieses Sicherungselement wird vor der Verwendung entfernt.

Dem Fachmann ist klar, dass die Form der Flansche grundsätzlich in verschiedener Weise ausgebildet sein kann. Insbesondere könnte auch genau ein umlaufender Flansch vorgesehen sein.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen eine schematische Darstellung eines Querschnitts durch die Vorrichtung:
- Fig. 1: bei in den Körper eingeführter Kanüle, vor dem Einfahren des sekundären Stössels;
- Fig. 2: nach dem Einfahren des sekundären Stössels, bei deaktivierter erster Verriegelungsvorrichtung, vor dem Zurückfahren des Spritzenkörpers;
- Fig. 3: bei zurückgefahrenem Spritzenkörper und deaktivierter zweiten Verriegelungsvorrichtung, vor dem Zurückfahren des primären Stössels;
- Fig. 4: im Endzustand.

Grundsätzlich sind in den Figuren gleiche Teile mit gleichen Bezugszeichen versehen.

### Wege zur Ausführung der Erfindung

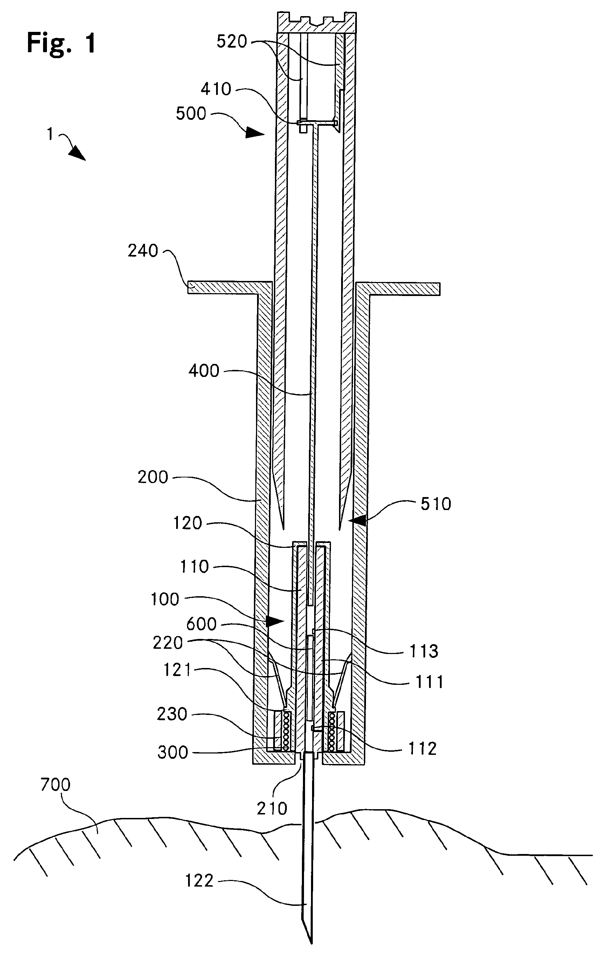

Die Figur 1 zeigt eine Ausführungsform einer erfindungsgemässen Vorrichtung 1 zur Injektion eines Feststoffes 600 in einen Körper 700.

Die Vorrichtung 1 umfasst ein Gehäuse 200, welches im Wesentlichen als einseitig geschlossener Kreiszylinder mit einer zentralen Öffnung 210 im Boden und einem am proximalen Ende radial nach aussen ragenden Flansch 240 als Griffelement, ausgebildet ist. Weiter umfasst das Gehäuse 200 eine am Boden angeordnete axial orientierte Führungshülse 230 mit einem grösseren Innendurchmesser als die Öffnung 210, in welcher eine Spiralfeder 300 koaxial geführt ist, welche in Figur 1 komprimiert ist. Weiter umfasst das Gehäuse 200 in einem distalen Bereich an der Innenwand zwei gegenüberliegende Zungen 220, welche federnd ausgeführt sind und im Ruhezustand in distaler Richtung nach innen verschwenkt sind.

Die Vorrichtung 1 umfasst weiter einen Spritzenkörper 100, welcher zweiteilig, aus einer Spritzenkörperhülse 120 und einer in der Spritzenkörperhülse 120 angeordneten Rückhaltevorrichtung 110, aufgebaut ist. Die Rückhaltevorrichtung 110 ist mit einer Kanüle 122 verbunden, wobei die durchgängige Öffnung der Rückhaltevorrichtung mit einem Durchgang der Kanüle 122 kommuniziert.

Die Rückhaltevorrichtung 110 ist innerhalb der Spritzenkörperhülse 120 angeordnet und befestigt. Dazu umfasst die Spritzenkörperhülse 120 proximal einen radial nach innen ragenden Flansch, welcher als Anschlag für die Rückhaltevorrichtung 110 dient. Die Spritzenkörperhülse 120 ist im Wesentlichen rotationssymmetrisch ausgebildet und umfasst aussenseitig ein radial nach aussen ragendes Rastelement 121, welches als umlaufender Flansch ausgebildet ist. Die distale Seite des Rastelements 121 steht in Kontakt mit der komprimierten Spiralfeder 300, die proximale Seite steht in Kontakt mit den Zungen 220. Durch die Zungen 220 wird damit eine erste Verriegelungsvorrichtung geschaffen. Die Rückhaltevorrichtung 110 ist im Wesentlichen als Hohlzylinder mit einer durchgängigen Öffnung ausgebildet und umfasst ein längliches Element 111, welches als U-förmige Ausnehmung im Hohlzylinder ausgebildet ist. Das längliche Element 111 umfasst radial nach innen ragenden Nasen 112, 113, wobei eine Nase 112 distal und eine weitere Nase 113 proximal am länglichen Element 111 angeformt ist. Zwischen den beiden Nasen 112, 113 ist vor der Injektion der Feststoff 600 gehalten. Das längliche Element 111 ist federnd ausgebildet, wobei die Nasen 112, 113 in einem Ruhezustand in die durchgängige Öffnung hineinragen. Wird nun der primäre Stössel 400 in die Rückhaltevorrichtung 110 eingeführt, so kontaktiert dieser vorerst die proximale Nase 113 und treibt diese, zusammen mit dem länglichen Element 111 radial nach aussen, womit die distale Nase 112 den Weg für den Feststoff 600 in die Kanüle 122 freigibt. Um die Verschwenkung des länglichen Elements 111 innerhalb der Spritzenkörperhülse 120 zu ermöglichen, weist ersteres zur Innenwand der Spritzenkörperhülse 120 einen Abstand auf. Dieser Abstand ist nicht dargestellt, kann aber auf verschiedene Weisen erreicht werden. Zum Beispiel kann das längliche Element 111 aussen geringfügig abgetragen sein, so dass dessen Materialstärke geringer ist als diejenige des restlichen Teils der Rückhaltevorrichtung 110. Anderseits kann auch die Spritzenkörperhülse 120 in diesem Bereich geringfügig abgetragen sein.

Der primäre Stössel 400 ist als kreiszylindrischer dünner, langer Stab ausgebildet und umfasst am proximalen Ende einen als radial orientierte Kreisscheibe ausgebildeten Anschlag 410.

Die Vorrichtung 1 umfasst weiter einen sekundären Stössel 500, welcher im Wesentlichen als einseitig geschlossener Kreiszylinder ausgebildet ist. In Richtung der Öffnung, das heisst in distaler Richtung, weist der sekundäre Stössel 500 eine aussenseitige stetige Verjüngung 510 auf, welche beim Einführen des sekundären Stössels 500 in das Gehäuse 200 die Zungen 220 kontaktieren und radial nach aussen drücken und die erste Verriegelungsvorrichtung entriegeln kann. Innenseitig umfasst der sekundäre Stössel 500 drei Zungen 520, wobei in den Figuren jeweils nur zwei ersichtlich sind, von denen eine in der Querschnittsebene der Darstellung liegt und daher schraffiert ist. Die Zungen 520 sind im Wesentlichen stabförmig ausgebildet und sind in einem proximalen Bereich mit der Innenwand und am proximalen Ende mit dem Bodenbereich des sekundären Stössels 500 verbunden. In einem distalen Bereich weisen die Zungen 520 eine radial geringere Dicke auf als im proximalen Bereich, womit ein Abstand zur Innenwand des sekundären Stössels 500 erhalten wird. Damit kann der distale Bereich der Zungen 520 federnd radial nach aussen geschwenkt werden. An der Innenseite weisen die Zungen 520 im distalen Bereich eine Kerbe auf, in welche der Anschlag 410 des primären Stössels 400 eingerastet ist, womit die zweite Verriegelungsvorrichtung geschaffen ist. Am distalen Ende, weisen die Zungen 520 an der Innenseite, anschliessend an die Kerben, in distaler Richtung orientierte, nach aussen gerichtete Abschrägungen auf. Diese Abschrägungen dienen als Kontaktbereich für den Spritzenkörper 100, womit letzterer die Zungen 520 radial nach aussen treiben und damit die zweite Verriegelungsvorrichtung deaktivieren kann.

Schliesslich zeigt die Figur 1 schematisch einen Körper 700, in welchen der Feststoff 700 zu injizieren ist.

Die Figur 1 zeigt die Vorrichtung 1 im Zustand vor der Injektion, wobei die Kanüle bereits in den Körper 700 eingeführt ist. Der Feststoff 600 ist zwischen den Nasen 112, 113 der Rückhaltevorrichtung 110 gehalten. Der primäre Stössel 400 ist über den Anschlag 410 in den Zungen 520 eingerastet und ist mit dem distalen Ende innerhalb der Rückhaltevorrichtung 110 des Spritzenkörpers, vor der proximalen Nase angeordnet.

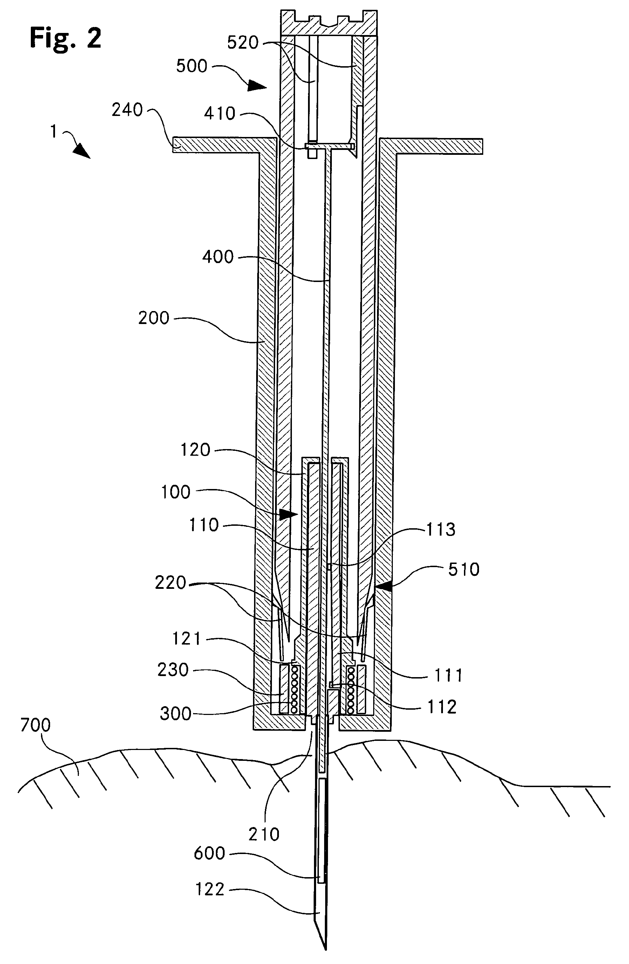

Die Figur 2 zeigt die Vorrichtung 1 im Wesentlichen gemäss Figur 1, wobei der sekundäre Stössel 500 vollständig eingefahren ist. Gleichzeitig ist dadurch auch der primäre Stössel 400 vollständig eingefahren und hat bereits den Feststoff 600 in die Kanüle 122, innerhalb des Körpers 700, überführt. Das längliche Element 111 der Rückhaltevorrichtung 110 ist radial nach aussen verschwenkt. Die Zungen 220 sind über den Bereich der Verjüngung 510 des sekundären Stössels 500 radial nach aussen verschwenkt und somit nicht mehr in Eingriff mit dem Rastelement 121 der Spritzenkörperhülse 120, womit die erste Verriegelungsvorrichtung entriegelt ist. Der Spritzenkörper 100 wird damit nicht mehr zurückgehalten und kann mittels der Spiralfeder 300 proximal Verfahren werden.

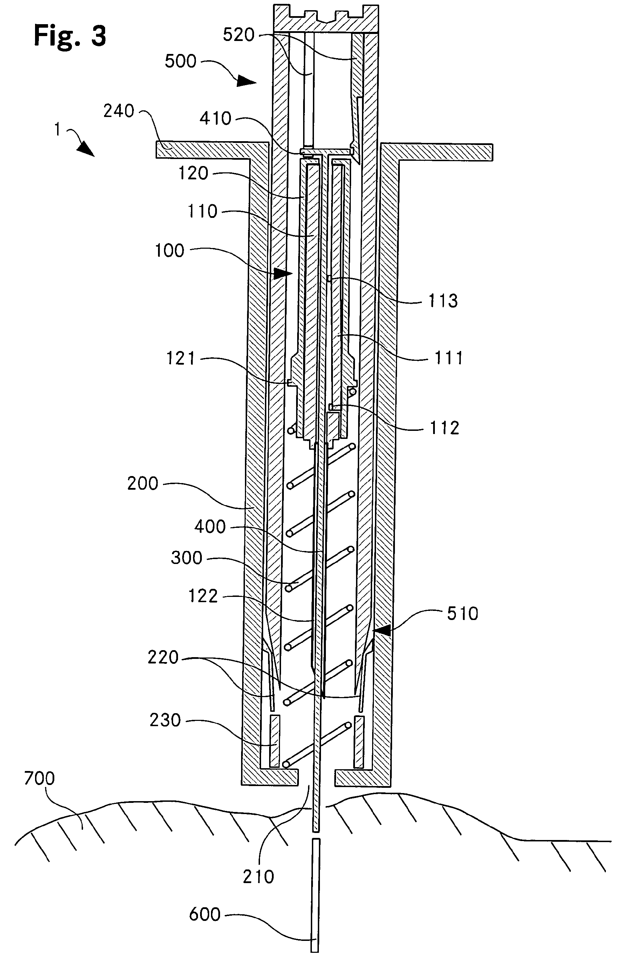

Die Figur 3 zeigt die Vorrichtung 1 im Wesentlichen gemäss Figur 2, wobei nun die Spiralfeder 300 teilweise entspannt ist. Der Spritzenkörper 100 ist zusammen mit der Kanüle 122, bei ortsfestem primärem Stössel 400, proximal Verfahren und kontaktiert mit seinem proximalen Ende die Abschrägungen der Zungen 520 und treibt diese radial nach aussen. Damit wird der Anschlag 410 von den Zungen 520 freigegeben, worauf der Spritzenkörper 100 den Anschlag 410 kontaktieren, und zusammen mit dem primären Stössel 400 proximal weiter verfahren kann. Der Feststoff 600 ist derweilen im Körper 700 platziert, der primäre Stössel 400 ragt noch in den Körper 700 hinein.

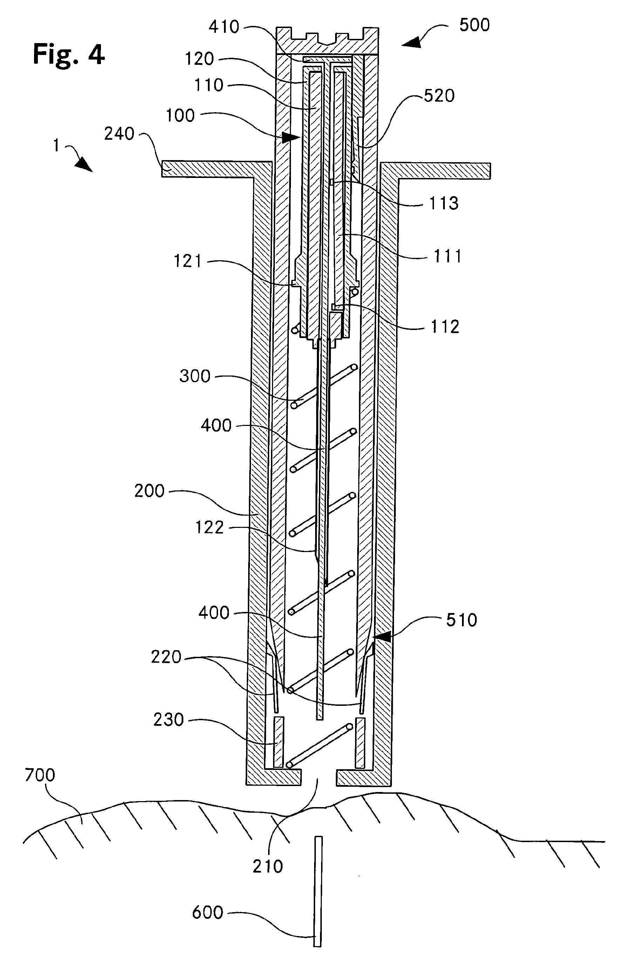

Die Figur 4 zeigt die Vorrichtung 1 im Wesentlichen gemäss Figur 3, in seinem Endzustand nach der Injektion. Die Spiralfeder 300 hat nun den Spritzenkörper 100 mit der Kanüle 122 zusammen mit dem primären Stössel 400 bis an den Anschlag des sekundären Stössels 500 geführt, womit nun die distalen, kontaminierten Bereiche der Kanüle 122 und des primären Stössels 400 innerhalb des Gehäuses 200 liegen.

Das Gehäuse 200 kann in Varianten auch die Führungshülse 230 umfassen, insbesondere einstückig mit der Führungshülse ausgebildet sein. Entsprechend können auch die Zungen 520 einstückig mit dem sekundären Stössel 500 ausgebildet sein.

Zusammenfassend ist festzustellen, dass erfindungsgemäss eine Vorrichtung 1 zur Injektion eines Feststoffes 600 geschaffen wird, welche sich durch einen besonders einfachen Aufbau wie auch durch eine einfache Bedienung auszeichnet.

## Patentansprüche

1. Vorrichtung (1) zum Injizieren eines Feststoffes (600) in einen menschlichen oder tierischen Körper, insbesondere eine Spritze für die Injizierung eines Feststoffmedikaments, umfassend:
a) einen in einem Gehäuse (200) verfahrbar gelagerten Spritzenkörper (100), verbunden mit einer Kanüle (122); wobei
b) der Spritzenkörper (100) in proximaler Richtung mit einer Federkraft beaufschlagt ist, wobei die Bewegung des Spritzenkörpers (100) in proximaler Richtung mittels einer ersten, mit dem Spritzenkörper (100) zusammenwirkenden Verriegelungsvorrichtung (121, 220) entriegelbar ist;
c) einen primären Stössel (400), welcher im Spritzenkörper (100) und in der Kanüle verfahrbar ist; wobei
d) der primäre Stössel (400) einen Anschlag (410) umfasst, welcher mit dem Spritzenkörper (100) zusammenwirken kann, wobei der Anschlag (410) in einem Zustand unmittelbar vor der Entriegelung der ersten Verriegelungsvorrichtung (121, 220) vom Spritzenkörper (100) proximal beabstandet ist; und wobei
e) über den Anschlag (410) des primären Stössels (400) derselbe durch den Spritzenkörper (100) bei entriegelter ersten Verriegelungsvorrichtung (121, 220) proximal zurückgefahren werden kann;
**dadurch gekennzeichnet, dass**
f) die erste Verriegelungsvorrichtung (121, 220) durch ein an der Vorrichtung (1) proximal angeordnetes Betätigungselement (500) deaktivierbar ist.

2. Vorrichtung (1) nach Anspruch 1, **dadurch gekennzeichnet, dass** vor der Verwendung der Vorrichtung (1) der Feststoff (600) im Spritzenkörper (100) gehalten und mittels des primären Stössels (400) in die Kanüle (122) überführbar ist.

3. Vorrichtung (1) nach Anspruch 1 oder 2, **dadurch gekennzeichnet, dass** die erste Verriegelungsvorrichtung (121, 220) ein federndes Element (220) und ein damit zusammenwirkendes starres Rastelement (121) umfasst, welche an einer Aussenseite des Spritzenkörpers (100) und an der Innenseite des Gehäuses (200) so angeordnet sind, dass sie in verriegeltem Zustand ineinander greifen.

4. Vorrichtung (1) nach Anspruch 3, **dadurch gekennzeichnet, dass** das federnde Element als längliche, in verriegeltem Zustand bezüglich einer Längsachse verschwenkte Zunge (220) ausgebildet ist, welche durch eine radial beaufschlagte Kraft im Wesentlichen in die axiale Richtung verschwenkbar ist, wodurch die erste Verriegelungsvorrichtung (121, 220) entriegelt werden kann.

5. Vorrichtung (1) nach Anspruch 3 oder 4, **dadurch gekennzeichnet, dass** das federnde Element (220) am Gehäuse (200) angeordnet ist.

6. Vorrichtung (1) nach einem der Ansprüche 3 bis 5, **dadurch gekennzeichnet, dass** das starre Rastelement (121) als vorstehende Nase ausgebildet, insbesondere am Spritzenkörper (100) angeformt ist.

7. Vorrichtung (1) nach einem der Ansprüche 1 bis 6, **dadurch gekennzeichnet, dass** bezüglich einer Längsachse mehrere symmetrisch angeordnete, insbesondere zwei gegenüberliegende erste Verriegelungsvorrichtungen (121, 220) vorgesehen sind.

8. Vorrichtung (1) nach einem der Ansprüche 1 bis 7, **dadurch gekennzeichnet, dass** die erste Verriegelungsvorrichtung (121, 220) mittels eines als sekundären Stössel (500) ausgebildeten Betätigungselements deaktivierbar ist.

9. Vorrichtung (1) nach Anspruch 8, **dadurch gekennzeichnet, dass** der sekundäre Stössel (500) als proximal geschlossener Hohlzylinder ausgebildet ist, welcher im Gehäuse (200) verfahrbar gelagert ist.

10. Vorrichtung (1) nach Anspruch 8 oder 9 und einem der Ansprüche 3 bis 7, **dadurch gekennzeichnet, dass** die erste Verriegelungsvorrichtung (121, 220) durch Kontaktierung des federnden Elements (220) durch den in im Gehäuse (200) eingefahrenem Zustand befindlichen sekundären Stössel (500) entriegelbar ist.

11. Vorrichtung (1) nach Anspruch 10, **dadurch gekennzeichnet, dass** der sekundäre Stössel (500) an einem distalen Ende in distaler Richtung eine kontinuierlich zunehmende Verjüngung (510) aufweist, womit in eingefahrenem Zustand das federnde Element (220) radial nach aussen geführt ist.

12. Vorrichtung (1) nach einem der Ansprüche 8 bis 11, **dadurch gekennzeichnet, dass** der sekundäre Stössel (500) lösbar mit dem primären Stössel (400) verbunden ist.

13. Vorrichtung (1) nach einem der Ansprüche 8 bis 12, **dadurch gekennzeichnet, dass** der sekundäre Stössel (500) innenseitig, im proximalen Bereich federnde Rasthaken (520) aufweist, welche als zweite Verriegelungsvorrichtung (410, 520), zum Verriegeln des Anschlages (410) des primären Stössels (400), ausgebildet sind.

14. Vorrichtung (1) nach Anspruch 13, **dadurch gekennzeichnet, dass** die zweite Verriegelungsvorrichtung (410, 520) durch den Spritzenkörper (100) entriegelbar ist.

15. Vorrichtung (1) nach Anspruch 13 oder 14, **dadurch gekennzeichnet, dass**
a) der Anschlag (410) als ein radial vom primären Stössel (400) abstehendes Element, insbesondere als radial orientierte Kreisscheibe, ausgebildet ist;
b) die federnden Rasthaken (520) als axial orientierte und radial verschwenkbare Zungen ausgebildet sind, welche innenseitig Ausnehmungen zur Aufnahme des Anschlages (410) aufweisen; wobei
c) die radial verschwenkbaren Zungen (520) durch den Spritzenkörper (100) radial nach aussen treibbar sind, womit die zweite Verriegelungsvorrichtung (410, 520) gelöst werden kann.

16. Vorrichtung (1) nach einem der Ansprüche 1 bis 15, **dadurch gekennzeichnet, dass** das Gehäuse (200) in einem proximalen Bereich einen umlaufenden Flansch (240) oder zwei gegenüberliegende radial nach aussen ragende Griffelemente (240) umfasst.
